# EUROPEAN PATENT SPECIFICATION

(11) **EP 0 925 795 B1**
(45) Date of publication and mention of the grant of the patent: **16.10.2002**
(21) Application number: 97926015.5
(22) Date of filing: 20.06.1997
(51) Int. Cl.: A61L 24/00, C09J 4/00, C07C 69/587

(54) **NEW CYANOACRYLIC-BASED ADHESIVE FORMULATIONS, PREPARATION PROCESS AND APPLICATIONS**
NEU KLEBSTOFFE AUF CYANOACRYLATBASIS, VERFAHREN ZU DEREN HERSTELLUNG UND VERWENDUNGEN
NOUVELLES FORMULATIONS ADHESIVES A BASE DE CYANOACRYLIQUE, PROCEDE DE PREPARATION, ET APPLICATIONS

(30) Priority: 21.06.1996 ES 9601398
(43) Date of publication of application: 30.06.1999
(73) Proprietor: UNIVERSIDAD DE ALICANTE, 03690 San Vicente del Raspeig (ES)
(72) Inventor: MARTIN MARTINEZ, José Miguel, E-03540 Playa de San Juan (ES); MAHIQUES BUJANDA, Maria del Mar, E-03540 Playa de San Juan (ES); ALIO Y SANZ, Jorge Luciano, E-03540 Playa de San Juan (ES); MULET HOMS, Maria Emilia, E-03016 Alicante (ES); FOUAD SAKLA, Heni, E-03560 El Campello (ES)
(74) Representative: Simpson, Paul Christopher
(86) International application number: ES9700161
(87) International publication number: WO97049436

## Description

The present invention fits within the technical field of the adhesives used to replace the traditional sutures in surgery.

More specifically, the present invention provides new adhesive alkyl cyanoacrylic formulations and semi-synthetic acrylic derivatives to replace sutures in surgery, which are specially apt for eye surgery.

### PRIOR ART OF THE INVENTION

Nowadays attempts to replace traditional sutures in the field of surgery by adhesive joining techniques are numerous. The purpose is multiple, since, on the one hand, an attempt is made to save time in the sealing of wounds and incisions. On the other hand, an attempt is made to avoid the additional traumatism entailed in sutures and finally, by means of techniques based on adhesion phenomena, a more uniform tissular union, where the pulling forces, that tend to separate the joined areas are distributed homogeneously, is pursued.

Although the use of adhesives in medicine is restricted to limit s ituations w here sutures a re n ot possible or convenient, the future of adhesives in surgery is promising.

As it has already been mentioned, adhesion techniques have been used in the joining of tissues sectioned in surface wounds, in the joining of bone prosthesis, in kidney and lung surgery in animals and in middle ear surgery, among others. In all cases notable improvements are achieved when adhesive unions are used instead of sutures since:
- No deformations are produced in the wound once the adhesive union has been carried out.
- The stresses are homogeneously distributed.
- The joining process is rapid.
- The union is flexible and allows inside areas of the
wound which would not be accessible by mechanical means to be filled.

Once the adhesive union has been carried out, the excess adhesive peels off the healed wound.

Nonetheless, adhesive unions of biological tissues have, up to now, inconveniences derived from the toxicity of the adhesives and from the formation of occlusions inside the tissues. Additionally, in the case of adhesives that do not have said toxicity, the adhesive unions that they produce are not strong enough to maintain joined the tissular substrates in which there are pulling forces opposite said unions.

In the field of eye surgery, the use of adhesives has permitted sutures to be replaced in cornea and retina surgery, in the following clinical applications:
- Sealing of traumatic perforations and ulcers on the cornea.
- Union of artificial membranes to the anterior surface of the cornea (artificial epithelium) and to the posterior surface (artificial endothelium).
- Adhesives in prostheses that penetrate in the cornea.
- Sealing of scleral perforations.
- Scleral loops without sutures.

It is possible to make a classification of the adhesives used in this type of surgery based on their origin, in such a way that natural adhesives are distinguished from synthetic adhesives.

### ADHESIVES OF BIOLOGICAL ORIGIN

### FIBRIN ADHESIVES

In the presence of calcium (II)ions, fibrinogen and factor XIII (fibrin stabilizing factor) are activated with thrombin to produce a stable fibrin clot. The fibrin by itself adheres to the collagen and factor XIII stimulates collagen synthesis, activating fibroblasts. Application of the fibrin adhesive on wounds allows restoration of the structural properties of the wound, as well as stimulation of the healing of said wound by its own components of the adhesive.

Present-day methods for the preparation of adhesives of this type have developed the inclusion of a large variety of proteins and factors implied in coagulation, along with thrombin.

The adhesive is generally applied by means of a double injection system that isolates the fibrinogen and the coagulation factors from the thrombin and aprotinin until the application thereof is carried out.

On the other hand, due to the existence of acquired human immune deficiency virus (HIV) and other blood contaminating products, methods to obtain said components as of the patient's own blood are being developed.

The joining forces of the fibrin adhesives are rather reduced, which limits, as it has already been mentioned, use thereof as structural adhesives. However, their rapid dissolution and absorption in vivo are factors to be considered when selecting an adhesive for surgical purposes.

Fibrin bioadhesives have been used in different fields of ophthalmology for more than 10 years. Some of the applications include retina, eyelid, lachrymal system, cornea ulceration surgery, as well as cataract surgery and surgery of perforations of the anterior and posterior capsule after undergoing perforating traumatisms of the crystalline lens.

In another type of surgery, such as the case of strabismus surgery, wherein insertion of a specific muscle in a corrected position on the sclera is required, fibrin adhesives are inadequate precisely due to the scarce adhesive force that they produce.

### SYNTHETIC ADHESIVES

### CYANOACRYLATES

Cyanoacrylate adhesives have been studied and tested in numerous field of surgery. Document FR-A-2 022 366 describes an adhesive composition comprising **methyl-2-cyanoacrylate and dimethyl methylenemalonate** or higher esters of 2-cyanoacrylic acid. Document US-A-3 940 362 is concerned with an adhesive composition comprising an ester of **2-cyanoacrylic acid** and a cross linking agent which is represented by a difunctional **ester of (meth)acrylic acid.** In eye surgery, there are numerous compounds of this type that have been analyzed and their applications have been evaluated by means of a broad number of surgical techniques.

In accordance with clinical and histological observations of tissular tolerance in rabbit eyes, it has been concluded that the best tolerated cyanoacrylate adhesives are n-decyl, n-octyl, n-heptyl, n-hexyl, n-butyl and isobutyl cyanoacrylate, somewhat worse tolerated is β-β-β-trifluoroisopropyl-2-cyanocrylate and the worst tolerated is methyl-2-cyanoacrylate. By means of sealing of corneal lacerations and perforations in rabbit eye by application of butyl, heptyl or octyl-2-cyanoacrylate, it has been demonstrated by post-operative evaluation (some weeks later) that the adhesive remained fixed on the tissues enough time for tissular healing to take place, without appreciable neovascularization of the cornea. Relevant tissular infiltration processes in eye membrane transplants have not been confirmed either in cyanoacrylate derivatives of this type.

However, methyl-2-cyanoacrylate has irritative processes when it is applied subconjunctivally to the sclera. Therefore, its use poses problems of irritation of eye tissues with marked inflammatory reactions, and handling thereof is problematical upon reticulating, likewise, on surgical instruments.

Among all the analogues described up to now, the most ideal ones for eye surgery purposes turned out to be isobutyl and butyl derivatives. However, these derivatives have some limitations not overcome up to now:
1) High reactivity against wet surroundings or substrates.
2) Needle-shape reticulation.
3) Opaque appearance of the product once it has polymerized.

Hence, use thereof in cornea lesions or surgery is limited, as well as in all those sealings of important areas that in turn require an extensive area to be joined by means of adhesives.

Finally, reference should be made to the long amount of time that these adhesives take in peeling off tissues. Two and three months after being instilled in experimental rabbit eyes, significant amounts of the same still remained, which delays the natural healing process.

This specification describes the preparation of a new series of cyanoacrylic adhesives, in whose formulation acrylic acids not described up until now are included, and which basically, have shown their effectiveness in the surgical union of different eye muscles to the sclera, also having, an optimal biological tolerance.

### DETAILED DESCRIPTION OF THE INVENTION

Just as its title indicates, the present invention refers to new adhesive cyanoacrylic formulations, process for preparation thereof and applications thereof in surgery, and especially in eye surgery.

The formulations of the invention are comprised of:
a) Alkyl cyanoacrylates
b) Alkyl carboxyacrylates, in a proportion of 10 to 30% by volume with regard to component (a).

Component (b) is comprised of new acrylic derivatives obtained semi-synthetically, which also form part of the present invention.

The starting materials to obtain the carboxyacrylic derivatives are the corresponding cyanoacrylic analogues. The reaction can be summarized as: 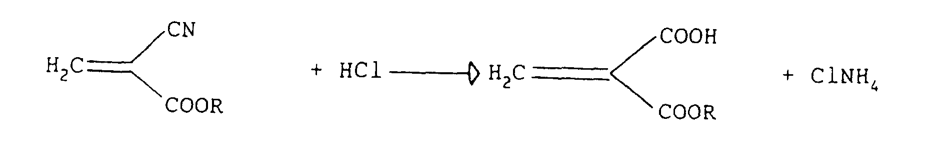 wherein R represents the C₁-C₈ alkyl residue.

The alkyl carboxylates are prepared by adding concentrated hydrochloric acid in an equimolar proportion, in an inert argon or nitrogen atmosphere and with electromagnetic stirring. When the adding of the acid ends, the mixture is heated to temperatures between 60 and 80° C for a variable amount of time (15-50 minutes).

Once the hydrolysis reaction has completed, the reaction product is extracted from the glass flask and is handled rapidly in an environment of low relative humidity. The treatment operations include:
1. Dilution of the reacting mixture with an organic non-chlorinated solvent of intermediate polarity.
2. Addition of a small volume of distilled water.
3. Extraction of the organic phase.
4. Washing of the organic phase with water, drying of the same with anhydrous sodium sulfate. Filtration and distillation of the solvent in vacuo.

In this way, a transparent, practically colorless liquid product with a viscosity slightly higher than the starting product is obtained and this product corresponds to the carboxyacrylic derivative.

The yield of the reaction depending on the starting cyanoacrylate is between 85 and 92%.

The characterization of the obtained products is done by techniques of IR spectroscopy and nuclear magnetic resonance of ¹H and ¹³C. The assignations corresponding to the functional groups and most relevant structural fragments of this new series of carboxyacrylic derivatives, respectively, are included in tables 1 and 2. In both tables the assignations are included as intervals given that reference is not made to a single substance, but rather they are valid for all analogues of the series.

**Table 1:** Assignations of the IR bands of the main groups of some carboxyacrylic derivatives.

| **Functional group** | **Absorbency/cm**^{**1**} |
|---|---|
| -COOH | 3550-2500 |
| | 1800-1650 |
| -COOR | 1790-1650 |
| | 1330-1050 |
| H₂C-C- | 3040-3010 |
| | 1690-1610 |
| | 990-900 |

**Table 2:** Assignations of the indications of NMR¹H and ¹³C of the common structural fragment of the carboxyacrylic derivatives series: 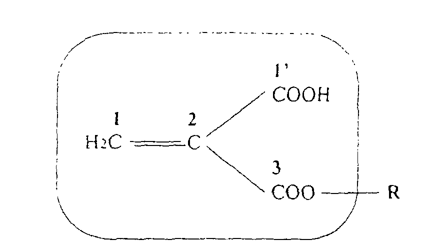 wherein the numbering has been carried out by choosing acrylic acid as the nomenclature base.

| ¹³C | displacement ppm | ¹H | displacement ppm |
|---|---|---|---|
| C-1 | 6.8-6.3 | H-1(2) | 7.1-6.3 |
| C-2 | 141-125 | ----- | ------- |
| C-3 | 185-175 | ----- | ------- |
| C-1' | 171-169 | ----- | ------- |

Preparation of the adhesive mixture is carried out at the latest one hour before being applied to live tissues. It has been verified that for longer times, the adhesive properties of the product are modified, since the adhesive capacity reduces, and with this, the strength of the joining to tissues (as of one hour, anionic polymerization of the two monomers that constitute the mixture, begins to be considerable).

Therefore, a useful time of one hour for the product already prepared is estimated.

It proves to be convenient to store the two acrylic compounds in independent containers, protected against exposure to light and moisture, the ideal temperature range for storage being between 5 and 15° C.

The cyanoacrylate mixture with 10-30% by volume of alkyl carboxyacrylate is preferably made in a glass or polyethylene container.

The miscibility of the two monomers that are used in the adhesive formulation proves to be optimal, achieving a single phase.

The new formulations of the present invention provide clear advantages over traditional surgical sutures, which may be summarized in the following points:
1.- Rapid sealing is achieved and this reduces surgical cost and time.
2.- Application of these products as well as the handling thereof prove to be simple as they can be packaged in convenient application systems.
3.- The products do not tend to undergo microbial contamination and therefore, sterility control thereof is easy.
4.- They are capable of closing tissues in the presence of biological fluids.
5.- They produce highly effective unions, even in considerably long incisions.
6.- They have a tensile joining force similar or greater than that of the tissues that are joined.
7.- They make a very strong union in a short amount of time (less than 1 minute).
8.- They maintain the strength of the adhesive union until the normal healing process concludes without inhibiting it.
9.- They do not cause irritation or emission of heat upon being instilled upon biological tissues.

These advantages observed in generally surgery convert in eye surgery, into the solving of the problem of joining extrinsic eye muscles, where sutures prove to be complex in most cases. Highly effective, rapid, persistent unions are achieved without interfering in the healing process.

Clinical tests carried out on experimental animals have evidenced the effectiveness of these new products in the above mentioned points, and likewise, it has been proven that they do not have necrotic or toxic effects, their macro and microscopic biological tolerance being very similar to that demonstrated by sutures, one week and one month after surgery.

The adhesive formulations thus obtained are provided with physical, chemical, viscoelastic and surface properties that make them more suitable for application thereof in eye surgery, with respect to the (synthetic or biological) adhesives that have been used or are being used nowadays. These improved properties and characteristics correspond to:
1. Good wetting capacity of the tissular substrate.
2. Suitable reticulation time of the adhesive product.
3. Formation of considerably strong adhesive unions.
4. Good tissular tolerance.
5. Optimal rigidity and transparency in the polymerized product.
6. Modifiable degradation and/or elimination time of the product.

Using different proportions of both compounds, always in the above mentioned suitable range of effectiveness, an important modification and modulation of the adhesive properties of the mixture are obtained, so that:
1. The viscosity of the formulation is controlled.
2. The time needed so that tissular adhesion takes place is modified.
3. The strength of the adhesive union is reinforced, or on the contrary, reduced.
4. The mechanical rigidity of the polymerized product is increased or decreased.
5. The time which the adhesive applied to the tissues takes to peel off is controlled.

The technical advantages that the formulations of the present invention offer with respect to the adhesives used up to now may be summarized in the following points:
1. Their reticulation is rapid enough but controlled, and therefore, no detectable alteration of the tissues involved in the adhesive union is produced.
2. The adhesion capacity of the new compounds allows the tissues to be kept firmly together during the entire healing process.
3. The adhesive reticulated on the tissues has a transparent appearance, its degree of rigidity being acceptable and therefore, those tissues that surround or rub against the adhesive union are not harmed.

When tissular union with the formulations of the invention is carried out, the most important factors to be considered are the water content of the tissues involved in the adhesive union, as well as the amount and proportions of the components in the adhesive mixture.

It has been experimentally proven that the new adhesive formulations described in this specification, are adequately tolerated by eye tissues such as the conjunctiva and Tenon's capsule, sclera and muscles. However, excessive wetting of the area by lacrimal liquid, circulating blood, or else, by the use of eye drops during the course of the operation should always be avoided. These precautions are necessary, at least, during the moment in which the adhesive product is being applied and during the time needed for reticulation thereof to take place. In any case, it has been verified that simple rubbing of the tissues with a hemostat suffices to avoid excess water.

With regard to the amount of adhesive that must be used, it is suggested that it be the minimum amount in order to achieve suitable wetting of the tissues or tissular areas to be adhered. Larger amounts, or an excess of adhesive, implies a two-fold problem: on the one hand, adhesion faults are produced, and on the other hand, correct approach of the tissues, necessary for the healing process to take place, is prevented.

### EMBODIMENTS OF THE INVENTION

The present invention is additionally illustrated by the following Examples, which do not intend to limit the scope of the same.

### EXAMPLE 1

### Synthesis and structural elucidation of the ethyl carboxylate

18 g. (0.144 mol) of ethyl cyanoacrylate are added to a glass flask with two mouths and a 250 ml. capacity under a constant nitrogen gas (2 bar) supply. A reflux refrigerant, an addition funnel containing 14.4 ml (0.173 mol) of concentrated hydrochloric acid and heating and electromagnetic stirring equipment are then coupled to the system.

The hydrochloric acid is added drop by drop from the addition funnel. When this has ended, heat is applied to the mixture by means of a silicone bath. It is observed that once the hydrochloric acid has been added, the mixture acquires a less transparent appearance due to the ammonium chloride that disperses in the center of the reaction. When the temperature that is reached in the system approaches 80° C, the reaction conditions are maintained for another 25 minutes.

Afterwards, the flask is left to cool to room temperature by moving it away from the heat source and some 100 ml. of distilled water are added, forming an emulsion. Likewise, some 50 ml. of ethyl acetate are added and this is all taken to a decantation funnel where the aqueous phase is separated and discarded.

The organic phase that remains in the funnel is washed successively by addition of small volumes of water, then dried over anhydrous sodium sulfate for at least 30 minutes. The ethyl acetate is filtered and distilled at reduced pressure, obtaining ethyl 2-carboxyacrylate.

The yield of the process is 91%.

Ethyl carboxyacrylate is a transparent and practically colorless liquid product at room temperature, whose IR spectrum has absorption bands assignable to the groups: carboxylic acid (3468, 1690 cm⁻¹), ester (1733 cm⁻¹) and olefin methylene (3075, 1614 and 991 cm⁻¹).

Its NMR¹H spectrum shows two singlet indications at 7.1 and 6.7 ppm, assignable to the two methylene protons; a double intensity and quadruplet multiplicity indication at 4.61 ppm, corresponding to the two protons on C-1 of the ethyl residue; and finally, a triplet centered at 1.35 ppm, assignable to the other three alkyl protons of the molecule.

With regard to the NMR¹³C spectrum, there are six indications assignable to methyl carbon (18.9 ppm), two methylene carbons (132.2 and 30.2 ppm), and three quaternary carbons (185.2; 171.3 and 127.1 ppm).

All these spectroscopic data are in accordance with the proposed structure for ethyl 2-carboxyacrylate.

### EXAMPLE 2

### Adhesion in vivo of the top straight eye muscle to a new scleral area with cyanoacrylate and ethyl carboxyacrylate (20%)

The top or bottom straight eye muscle in a anesthetized rabbit is dissected in order to be later inserted in a new position on the sclera by applying the new adhesive mixture and following the procedure that is described hereinafter:

The conjunctival tissue is separated along with the Tenon's capsule, from the limbic area up to the straight muscle and a new position is located on the sclera, generally retroinserted some millimeters (4 mm., marked with India ink) with regard to the initial position. Upon this chosen scleral area, with the help of a small bistoury the tissue is gently scraped for the purpose of increasing the surface of contact with the adhesive and a drop of adhesive formulation is deposited through a 0.1 mm. diameter cannula. Immediately afterwards, the end of the dissected muscle is place on it. It is kept in the new position by exerting a slight pressure for a few seconds, whereby the correct union of the two tissues is achieved.

One waits a minute before any pulling after application of the adhesive in order to check that in effect the adhesion is firm, and to then reapply the conjunctiva along with the Tenon's capsule that are taken to their original position. A minimum amount of adhesive is deposited on the same point where separation of these tissues begun, adhering again to the limbic area.

## Claims

1. A cyanoacrylic adhesive formulation for biological tissues, the formulation comprising
a first component selected from C₁- C₈ alkyl cyanoacrylates; and
a second component selected from C₁-C₈ alkylcarboxylates in a proportion of 10-30vol.% with regard to the first component.

2. A formulation according to claim 1, wherein the first and the second component are separate before being applied.

3. A formulation according to claim 1 or 2, wherein the second component is ethylcarboxyacrylate.

4. A component for an adhesive formulation for biological tissues, the component comprising an alkyl carboxyacrylate of the general formula 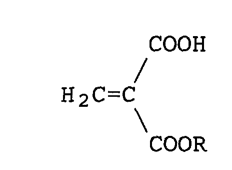 wherein R is and alkyl group of 1 to 8 carbon atoms.

5. A component according to claim 4, wherein R is an ethyl group.

6. A process for preparing an alkyl carboxyacrylate of the general formula 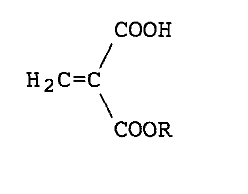 wherein R is an alkyl group of 1 to 8 carbon atoms, the process comprising
a first step of preparing a first reaction mixture by adding, with stirring in an inert atmosphere selected from a nitrogen or argon atmosphere, concentrated hydrochloric acid, to a structurally analogous cyanoacrylate of the formula 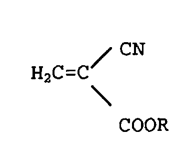 wherein R is an alkyl group of 1 to 8 carbon atoms, in an equimolar proportion;
a second step of heating the first reaction mixture to a temperature between 60ºC and 80ºC during 15-50 minutes until obtaining a second reaction mixture; and
a third step of isolating the alkyl carboxyacrylate from the second reaction mixture.

7. A process according to claim 6, wherein the third step comprises the operations of
diluting the second reaction mixture with an unchlorated organic solvent of intermediate polarity;
adding a small volume of distilled water to form a first liquid containing an organic phase and an aqueous phase; and
washing the organic phase with water, drying with anhydride sodium sulfate and distilling the organic solvent.

8. A process for preparing an adhesive cyanoacrylic formulation for biological tissues, the process comprising mixing a first component selected from C₁-C₈ alkyl cyanoacrylates with a second component selected from C₁-C₈ alkylcarboxylates in a proportion of 10-30vol.% with regard to the first component, not later than one hour before being applied to the biological tissues.

9. Use of an alkyl carboxyacrylate of the general formula 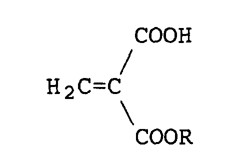 wherein R is and alkyl group of 1 to 8 carbon atoms, in the preparation of adhesive formulations for tissular sealing in surgery.

10. Use of an alkyl carboxyacrylate of the general formula 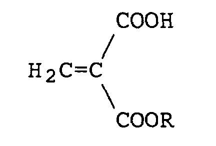 wherein R is and alkyl group of 1 to 8 carbon atoms, in the preparation of adhesive formulations for bonding eye muscles to the eye sclera.

## Patentansprüche

1. Eine Cyanoacrylatkleberformulierung für biologische Gewebe, wobei die Formulierung umfasst:
eine erste Komponente, die ausgewählt ist aus C₁-C₈-Alkylcyanoacrylaten und
eine zweite Komponente, die ausgewählt ist aus C₁-C₈-Alkylcarboxylaten, in einem Anteil von 10 bis 30 Volumen-%, bezogen auf die erste Komponente.

2. Eine Formulierung gemäß Anspruch 1, worin die erste und die zweite Komponente getrennt sind, bevor sie aufgetragen werden.

3. Eine Formulierung gemäß Anspruch 1 oder 2, worin die zweite Komponente Ethylcarboxyacrylat ist.

4. Eine Komponente für eine Kleberformulierung für biologische Gewebe, wobei die Komponente ein Alkylcarboxyacrylat der allgemeinen Formel 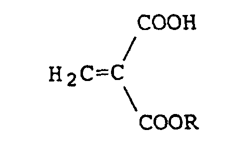 umfasst, worin R eine Alkylgruppe mit 1 bis 8 Kohlenstoffatomen ist.

5. Eine Komponente gemäß Anspruch 4, worin R eine Ethylgruppe ist.

6. Ein Verfahren zur Herstellung eines Alkylcarboxyacrylats der allgemeinen Formel 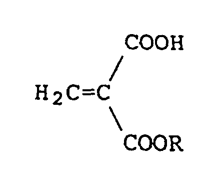 worin R eine Alkylgruppe mit 1 bis 8 Kohlenstoffatomen ist, wobei das Verfahren umfasst:
einen ersten Schritt der Herstellung einer ersten Reaktionsmischung durch Zugeben unter Rühren in einer inerten Atmosphäre, die ausgewählt ist aus einer Stickstoffoder Argonatmosphäre, von konzentrierter Salzsäure zu einem strukturell analogen Cyanoacrylat der Formel 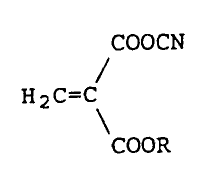 worin R eine Alkylgruppe mit 1 bis 8 Kohlenstoffatomen ist, in einem äquimolaren Verhältnis;
einen zweiten Schritt des Erwärmens der ersten Reaktionsmischung auf eine Temperatur zwischen 60°C und 80°C während 15 - 50 Minuten, bis eine zweite Reaktionsmischung erhalten wird; und
einen dritten Schritt des Isolierens des Alkylcarboxyacrylats aus der zweiten Reaktionsmischung.

7. Ein Verfahren gemäß Anspruch 6, worin der dritte Schritt die folgenden Arbeitsgänge umfasst:
Verdünnen der zweiten Reaktionsmischung mit einem unchlorierten organischen Lösungsmittel mittlerer Polarität;
Zugeben eines kleinen Volumens an destilliertem Wasser, um eine erste Flüssigkeit zu bilden, welche eine organische Phase und eine wässrige Phase enthält; und
Waschen der organischen Phase mit Wasser, Trocknen mit wasserfreiem Natriumsulfat und Destillieren des organischen Lösungsmittels.

8. Ein Verfahren zur Herstellung einer klebenden Cyanoacrylatformulierung für biologische Gewebe, wobei das Verfahren das Mischen einer ersten Komponente, die ausgewählt ist aus C₁-C₈-Alkylcyanoacrylaten, mit einer zweiten Komponente, die ausgewählt ist aus C₁-C₈-Alkylcarboxylaten, in einem Anteil von 10 bis 30 Volumen-%, bezogen auf die erste Komponente, nicht später als 1 Stunde, bevor diese auf die biologischen Gewebe aufgetragen wird, umfasst.

9. Verwendung eines Alkylcarboxyacrylats der allgemeinen Formel 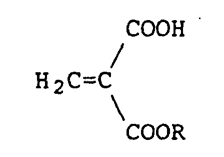 worin R eine Alkylgruppe mit 1 bis 8 Kohlenstoffatomen ist, bei der Herstellung von Kleberformulierungen zum Verschließen von Geweben bei Operationen.

10. Verwendung eines Alkylcarboxyacrylats der allgemeinen Formel 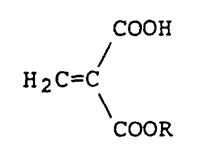 worin R eine Alkylgruppe mit 1 bis 8 Kohlenstoffatomen ist, bei der Herstellung von Kleberformulierungen zum Kleben von Augenmuskeln an die Lederhaut des Auges.

## Revendications

1. Formulation d'adhésif cyanoacrylique pour tissus biologiques, la formulation comprenant
un premier composant choisi parmi les cyanoacrylates d'alkyle en C₁-C₈ ; et
un second composant choisi parmi des carboxylates d'alkyle en C₁-C₈ en une proportion de 10-30 % vol. par rapport au premier composant.

2. Formulation selon la revendication 1, dans laquelle le premier et le second composants sont séparés avant d'être appliqués.

3. Formulation selon la revendication 1 ou 2, dans laquelle le second composant est le carboxylacrylate d'éthyle.

4. Composant pour une formulation d'adhésif pour tissus biologiques, le composant comprenant un carboxyacrylate d'alkyle de formule qénérale 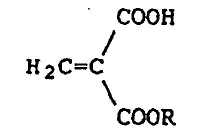 dans laquelle R représente un groupe alkyle de 1 à 8 atomes de carbone.

5. Composant selon la revendication 4, dans lequel R représente un groupe éthyle.

6. Procédé de préparation d'un carboxyacrylate d'alkyle de formule générale 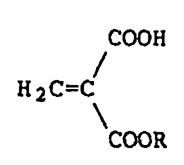 dans laquelle R représente un groupe alkyle de 1 à 8 atomes de carbone, le procédé comprenant
une première étape de préparation d'un premier mélange réactionnel par addition, sous agitation dans une atmosphère inerte choisie parmi une atmosphère d'azote ou d'argon, d'acide chlorhydrique concentré à un cyanoacrylate structurellement analogue de formule 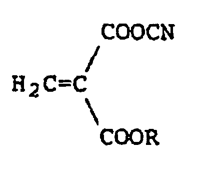 dans laquelle R représente un groupe alkyle de 1 à 8 atomes de carbone, en une proportion équimolaire ;
une seconde étape de chauffage du premier mélange réactionnel jusqu'à une température comprise entre 60 °C et 80 °C pendant 15-50 minutes jusqu'à obtention d'un second mélange réactionnel ; et
une troisième étape d'isolement du carboxyacrylate d'alkyle à partir du second mélange réactionnel.

7. Procédé selon la revendication 6, dans lequel la troisième étape comprend les opérations consistant
à diluer le second mélange réactionnel avec un solvant organique non chloré de polarité intermédiaire ;
à ajouter un petit volume d'eau distillée pour former un premier liquide contenant une phase organique et une phase aqueuse ; et
à laver la phase organique avec de l'eau, à sécher avec du sulfate de sodium anhydre et à distiller le solvant organique.

8. Procédé de préparation d'une formulation cyanoacrylique d'adhésif pour tissus biologiques, le procédé comprenant le mélange d'un premier composant choisi parmi les cyanoacrylates d'alkyle en C₁-C₈ avec un second composant choisi parmi des carboxylates d'alkyle en C₁-C₈ en une proportion de 10-30 % vol. par rapport au premier composant, pas plus d'une heure avant application aux tissus biologiques.

9. Utilisation d'un carboxyacrylate d'alkyle de formule générale 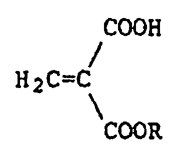 dans laquelle R représente un groupe alkyle de 1 à 8 atomes de carbone, dans la préparation de formulations d'adhésif pour scellement tissulaire en chirurgie.

10. Utilisation d'un carboxyacrylate d'alkyle de formule générale 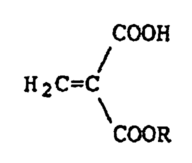 dans laquelle R représente un groupe alkyle de 1 à 8 atomes de carbone, dans la préparation de formulations d'adhésif pour attacher les muscles de l'oeil à la sclérotique de l'oeil.
